# EUROPEAN PATENT SPECIFICATION

(11) **EP 3 027 165 B1**
(45) Date of publication and mention of the grant of the patent: **17.11.2021**
(21) Application number: 14750926.9
(22) Date of filing: 30.07.2014
(51) Int. Cl.: A61K 6/887

(54) **IMPACT MODIFIED DENTURE BASE COMPOSITIONS**
SCHLAGMODIFIZIERTE ZAHNPROTHESEN-BASISZUSAMMENSETZUNGEN
COMPOSITIONS ANTICHOC POUR BASE DE PROTHÈSE

(30) Priority: 30.07.2013 US 201361859821 P
(43) Date of publication of application: 08.06.2016
(73) Proprietor: DENTSPLY SIRONA Inc., York, PA 17401-2991 (US)
(72) Inventor: SUN, Benjamin, Jiemin, York, PA 17402 (US); GHERGULESCU, Camelia, York, PA 17402 (US); YOUNG, Andrew, Dallastown, PA 17313 (US)
(74) Representative: Hartz, Nikolai
(86) International application number: PCT/US2014/048921
(87) International publication number: WO 2015/017556

(56) References cited:
- WO-A1-2006/053154
- WO-A1-2014/078537
- DE-A1- 19 617 876
- GB-A- 1 418 411
- US-A1- 2006 217 488
- None

## Description

### BACKGROUND OF THE INVENTION

This invention provides novel polymerizable rubber impact modified liquid compositions useful for a wide range of applications and method for the preparation of this liquid. Particular utility is found in the dental field where such compositions are highly suitable for the formation and construction of denture bases, and for other dental and prosthetic uses, such as denture baseplates, denture liners, denture repair, splints, orthodontic appliances, custom trays, veneer, crown and bridge, artificial teeth, repair for natural teeth, and teeth restorative fillings, etc. Specifically, the invention relates to polymerizable liquid compositions comprising rubber impact modifiers, monomers, and multifunctional crosslinking monomers or oligomers for said monomers, and optionally soluble or dispersible polymers or compounds which form liquid mixtures. These liquid mixtures are capable of being formed or molded with polymer powders, such as PMMA polymer powder/beads and caused to polymerize so as to provide articles possessing superior impact resistance, physical and physiochemical properties. Furthermore, excellent molding or flowable processability is useful in the production of a denture or a dental restorative material.

Denture base should exhibit certain desirable physical characteristics to be suitable for use and offer desirable benefits to patients. They should be dimensional stable for effective functioning, sufficient strength to withstand masticating stresses and resistant to abrasion and chipping during use. They also should be durable and stable to solvents, foods, water, cold and hot and maintain esthetics without discoloration. In addition, they should be esthetics to mimic natural dentition and gum with esthetically acceptable color, i.e., close to that of natural gum. The denture base should not wear or deform out of occlusion, and should be capable of being bonded firmly to artificial teeth. They should also be adjustable to ordinary means of physical shaping, grinding, and polishing. Denture base materials should give desirable handling characteristics to be suitable for the fabrication of denture base and offer desirable benefits to dental technicians. Dental base materials should be stable without discoloration and provide consistent handling properties during their shelf life.

Typically, denture bases are methacrylate-based acrylics, thermoplastic based or light curable resin based. In general, methacrylate-based acrylic denture bases are made out of dough from the blending of PMMA or modified PMMA polymer powders with MMA or modified MMA liquids. However, PMMA and MMA based denture bases have the disadvantage of being subject to brittle fracture due to the nature of PMMA. Rubber impact modified PMMA acrylics were used to improve their fracture toughness and impact strength. Rubber impact modified PMMA acrylics are typically made by incorporating MBS rubber particles. Of the presently available high impact acrylic compositions used for the construction of denture bases, all are composed of clear MMA based liquid containing polyfunctional moieties as crosslinkers and rubber impact modified acrylic polymer powders. While such high impact compositions are commonly in use in the construction of toughened denture bases, they nonetheless possess certain drawbacks. In general, these rubber impact modified polymer powders are not suitable for use in cold cure acrylics due to the undesirable mixing results and performance, such as the difficulty to remove entrapped tiny air bubbles. Typical rubber impact modified polymer powders are not very color stable and their powders show yellow shift over a long period of time or have accelerated yellow shift at an elevated temperature, which resulted in off shade dentures. When the polymer powders were stored for an extended period, color shift not only affected the shade accuracy of finished products but also made the repair of finished products difficult. They are prune to color changes under heat and UV irradiation. These rubber impact modified polymers are also more difficult to manufacture and cost more than standard PMMA. In addition, the dentures made from those MBS rubber based materials typically have the tendency to show color changes after use for a long period of time. Nevertheless, denture bases made of currently available impact modified acrylic compositions in general are adequate for impact resistant denture bases.

Various patents have claimed the impact resistant polymer compositions, which offer improved impact resistance due to the use of rubber impact modified polymer powders. There are few patents to claim high impact resistant denture base polymers, even though several commercially available denture base products are being sold for high impact resistant denture bases.

Takahashi et al., US Patent 5,283,286 disclosed a thermoplastic resin composition prepared by blending 50-95 wt% of a copolymer (A) of 10-75 wt% of -methyl styrene, 10-65 wt% of methyl methacrylate, 5-35 wt% of acrylonitrile and 0-20 wt% of a monomer copolymerizable therewith: and 5-50 wt% of a graft copolymer (B) comprising 50-85 wt parts of a butadiene-based rubber polymer and 15-50 wt parts of a vinyl monomer graft-polymerized onto the rubber polymer. The butadiene-based rubber polymer includes 50-98 wt% of butadiene, 2-50 wt% of an aromatic polycyclic vinyl monomer and 0-20 wt% of a monomer.

Kabs and Mueck (US Patent 7,173,093) disclosed impact-modified polymethyl methacrylate (PMMA) and a process for preparing the polymethyl methacrylate, and also articles which can be produced from the impact-modified PMMA.

Yamamoto et al., US Patent 5,182,332 disclosed a dental composition comprising a (meth)acrylic ester compound as the principal component and containing a polymerization initiator, which is characterized by having incorporated therein a grafted rubber obtained by polymerizing at least one ethylenically unsaturated monomer in the presence of a rubber. This dental composition can be cured to yield a cured product having excellent strength properties and, therefore, is particularly useful in applications such as molar restoration material and a denture base resin.

Kubota and Kusakai (US Patent 4,398,007) disclosed a denture base resin composition contains a liquid component obtained by incorporation of 0.5 to 20% by weight of a particular silane compound and 0.5 to 10% by weight of an unsaturated carboxylic acid into a methyl methacrylate monomer. The liquid component may be mixed with a methyl methacrylate polymer in a mixing ratio (by weight) of 1:3 to 3:1.

Okada and Tsukamoto (US patent 5,698,611) disclosed denture base relining resin comprises a powder component that is a methacrylate ester polymer powder with benzoyl peroxide added thereto and a liquid component that comprises a combination of methyl methacrylate, a methacrylate and a monomer having a specific structure with at least one compound selected from 4-dimethylaminobenzoate and 4,4'-dimethylaminobenzophenone, a specific tertiary amine and a polymerization inhibitor, all incorporated in said liquid component at specific ratios.

Kubo and Kazuhiko (US Patent 6,063,831) disclosed a method for using dental acrylic resins, the method comprising mixing a liquid monomer of methyl methacrylate with a polymer powder of methyl methacrylate, forming the mixture into an arbitrary shape by molding, filling, applying or other means, and then polymerizing and curing the formed mixture, wherein organic compounds selected from acryloyl morpholine, a coumarone resin, vinyl stearate, polyvinyl acetate, and alcoholic surfactants are previously added to the liquid monomer as a shrinkage controlling agent for lowering a curing shrinkage caused by the polymerization.

Kobayashi and Kumagai (US Patent 6,576,711) disclosed a resin material for denture base that is less in possibility to incorporate air bubbles into a mixed material, exhibits a proper elasticity. The resin material for denture base is of a two-liquid type, constructed by (a) a methacrylate or acrylate monomer having at least one unsaturated double bond; (b) one or two or more polymers selected from the group consisting of a methacrylate polymer, an acrylate polymer, a methacrylate/acrylate copolymer, a methacrylate/styrene copolymer, and an acrylate/styrene copolymer; and (c) a polymerization initiator, wherein a liquid A comprises the polymer (b), the monomer (a) having a solubility to the polymer (b) of 20% by weight or more, and the polymerization initiator (c); and a liquid B comprises the polymer (b) and the monomer (a) having a solubility to the polymer (b) of less than 20% by weight. When mixing the liquid A with the liquid B, the monomer (a) in the liquid A swells or dissolves the polymer (b) in the liquid B to increase its viscosity, whereby the mixture is polymerized and cured.

Tateosian, et al., US Patent 5,502,087 disclosed a dental prosthesis including polymeric material formed by heat curing a polymerizable composition having at least 5 percent by weight of at least one polymerizable monomer having at least one acrylic moiety and a gram molecular weight of at least 200. Preferably the polymerizable composition includes polymerizable compounds made up of at least 5 percent by weight of the acrylic monomer having a gram molecular weight of at least 300 and less than 5 percent by weight of volatile compounds and less than 2 percent by weight of low molecular weight acrylic compounds having a gram molecular weight of less than 200.

Rheinberger, et al., DE 196 17 876 A1, disclosed a polymerizable dental material with high impact resistance suitable for dentures. The material comprises impact modifiers comprising a polysiloxane graft copolymer with an elastomeric polysiloxane core and a shell of a non-elastomeric polymer.

Sun and Lichkus, WO 2006/053154 A1, disclosed wax-like polymeric dental materials for forming dentures, comprising oligomeric urethane (meth)acrylates and rubber modified compounds, such as a methacrylated butadiene styrene impact modifier.

Renz, et al., US 2006/217488 A1, disclosed materials for producing dental prostheses. Disclosed is a two-component prosthetic base material consisting of a liquid monomer component, and a powdered filler-containing component containing at least one bead polymer modified by an elastic phase. The bead polymer may be modified by, e.g., butadiene-styrene copolymer.

Sun et al., WO 2014/078537 A1, disclosed printable polymerizable materials to make dental products, comprising rubber impact modifiers, where the core-shell (core is silicone, shell is PMMA acrylic) rubber impact modifier does not dissolve but swells and forms a colloid at room temperature or at elevated temperature. The core-shell impact modifiers may comprise various polymeric materials, e.g., based on butadiene. In an example, a polymerizable dental material was prepared from a liquid mixture of about 20-30% by wt of an oligomer, about 45-55% by wt of methyl methacrylate, about 5-15 wt% of a polymer and about 3-10 wt% of a silicone-acrylic based rubber impact modifier.

### SUMMARY OF INVENTION

The object of this invention is to provide novel rubber impact modified liquid compositions which are useful in the construction of denture bases, artificial teeth, and other dental appliances, which lead to products having improved impact resistance and superior physical and esthetic characteristics.

The scope of the invention is defined by independent claim 1. Preferred embodiments are described by the dependent claims.

In one aspect, the present invention provides a composition comprising at least one polymerizable liquid component including at least one rubber impact modifier; at least one polymerization initiator, and at least one polymer component.

In another aspect, the present invention contemplates a dental composition comprising: a) a polymerizable liquid component including: (i) a core/shell rubber impact modifier; (ii) a crosslinking agent; (iii) at least one monomer; and (iv) at least one first initiator; b) a polymer powder component including: (i) at least one copolymer; and (ii) at least one second initiator; wherein the cured composition forms a material having a Kₘₐₓ of at least 1.8 MPa*m^{1/2} and a work of fracture of at least 700 J/m.

The present invention is further characterized by the following features: the at least one polymer component is a powder; the at least one polymerizable liquid component includes at least one monomer, at least one crosslinking agent for the monomer, and the at least one rubber impact modifier; the rubber impact modifier is a core-shell rubber impact modifier; the core-shell rubber impact modifier does not dissolve but swells and forms a colloid in the at least one polymerizable liquid component; the at least one monomer is selected from a group consisting of methyl acrylate, ethyl methacrylate, isobutyl methacrylate, cyclohexyl methacrylate, isobornyl methacrylate, isobornyl acrylate, allyl methacrylate; the at least one crosslinking agent is selected from a group consisting of di- or poly-acrylates and methacrylates such as glycerol di(meth)acrylate, glycerol tri(meth)acrylate, ethyleneglycol di(meth)acrylate, diethyleneglycol di(meth)acrylate, triethyleneglycol dimethacrylate, tetraethylene glycol di(meth)acrylate, poly(ethylene glycol) di(meth)acrylate, poly(propylene glycol) di(meth)acrylate, 1,3-propanediol di(meth)acrylate, 1,3-propanediol di(meth)acrylate, 1,4-butanediol di(meth)acrylate, 1,3-butylene glycol di(meth)acrylate, trimethylolpropane tri(meth)acrylate, 1,2,4-butanetriol trimethacrylate, 1,4-cyclohexanediol diacrylate, 1,4-cyclohexanediol dimethacrylate, 1,6-hexanediol di(meth)acrylate, neopentyl glycol di(meth)acrylate, tris(2-hydroxy ethyl)isocyanurate diacrylate, tris(2-hydroxy ethyl)isocyanurate triacrylate, pentaerythritol tri(meth)acrylate, pentaerythritol tetra(meth)acrylate, pentaerythritol tetramethacrylate, sorbitol hexacrylate, 2,2-bis[4-(2-hydroxy-3-acryloyloxypropoxy)phenyl]propane; 2,2-bis[4-(2-hydroxy-3-methacryloyloxypropoxy)phenyl]propane (Bis-GMA); 2,2-bis[4-(acryloyloxy-ethoxy)phenyl]propane; 2,2-bis[4-(methacryloyloxy-ethoxy)phenyl]propane (or ethoxylated bisphenol A-dimethacrylate) (EBPADMA); urethane di(meth)acrylate (UDMA), such as urethane di(meth)acrylate derivative of (isocyanatomethyl)cyclohexane (e.g., 1,3-bis(isocyanatomethyl)cyclohexane), UCDPMAA of example 2, diurethane dimethacrylate (DUDMA), 4,13-dioxo-3,14 dioxa-5,12-diazahexadecane-1,16-diol diacrylate; 4,13-dioxo-3,14 dioxa-5,12-diazahexadecane-1,16-diol dimethacrylate; the reaction product of trimethyl 1,6-diisocyanatohexane and bisphenol A propoxylate and 2-hydroxyethyl methacrylate (TBDMA); the reaction product of 1,6 diisocyanatohexane and 2-hydroxyethyl methacrylate modified with water (HDIDMA); the reaction product of 1,6 diisocyanatohexane and 2-hydroxyethyl acrylate modified with water (HDIDA); polyurethane dimethacrylate (PUDMA); alkoxylated pentaerythritol tetraacrylate; polycarbonate dimethacrylate (PCDMA); the bis-acrylates and bis-methacrylates of polyethylene glycols; copolymerizable mixtures of acrylated monomers and acrylated oligomers and combinations thereof; the resultant composition forms a flowable liquid.
The invention may be further characterized by one or any combination of the following features: The at least one rubber impact modifier disperses evenly and maintains a homogeneous appearance in the at least one polymerizable liquid component; the cured composition forms a material having a Kₘₐₓ of at least 1.8 MPa*m^{1/2} and a work of fracture of at least 700 J/m; the cured composition forms a high impact material having a Kₘₐₓ of at least 2 MPa*m^{1/2} and a work of fracture of at least 900 J/m; the at least one impact modifier includes a core/shell impact modifier having greater than 30% by wt being a first polymeric core material that is encapsulated by a second polymeric shell material; the first polymeric core material and the second polymeric shell material include one or more polymers that are combined and/or reacted together (e.g., sequentially polymerized) or may be part of separate or same core/shell systems; the at least one monomer of the at least one polymerizable liquid component includes methyl methacrylate (MMA); the composition further comprising one or more additives is selected from the group consisting of at least one filler, an accelerator, an inhibitor, surfactant, and combinations thereof; the at least one filler is selected from the group consisting of fibers, glass particles and combination thereof); the at least one polymer powder component is selected from the group consisting of methyl methacrylate based polymers, copolymers, crosslinked polymers, plasticized polymers, rubber impact modified polymers and combinations thereof; or any combination thereof.

It should be appreciated that the above referenced aspects and examples are nonlimiting as others exist with the present invention, as shown and described herein. For example, any of the above mentioned aspects or features of the invention may be combined to form other unique configurations, within the scope of the appended claims.

### DETAILED DESCRIPTION OF THE INVENTION

This invention provides a novel alternative to the traditional use of rubber impact modified polymer powders through the use of rubber impact modified liquid system, where core-shell rubber impact modifier does not dissolve but swells and forms a colloid. In general, the novel rubber impact modified liquid compositions of this invention are useful for the formation, construction of denture bases, artificial teeth, veneers, crowns and bridges, dentures, dental appliance and prosthetics, etc. This invention provides a superior alternative to modify the impact resistance of the denture bases. The use of rubber impact modified liquids offers several advantages. By reducing or eliminating the need of more expensive rubber impact modified polymer powders, the rubber impact modified liquids can be manufactured in a shorter time at a lower cost. Moreover, the use of rubber impact modified liquids provides flexibility for mixing the liquid with powder, which allows the improvement of impact strength for several different denture base products with different handling properties since the liquids can be used with any kind of denture base polymer powders. In addition, the use of rubber impact modified liquids avoids the potential porosity issues associated with the use of rubber impact modified polymer powders in cold cure acrylics. Finally, the use of rubber impact modified liquids of this invention can reduce or eliminate the potential color stability issue typically related to denture base polymer powders which are MBS based rubber impact modified polymers.

In accordance with a preferred form of the present invention, novel rubber impact modified dental liquid compositions are provided, which may easily and conveniently be molded and polymerized with conventional acrylic polymer powders by known techniques into prosthetic appliances possessing chemical and physical properties which are significantly improved over those of conventional prior art acrylic dental appliances. Notably, dental appliances, such as, for example, various prosthetic denture bases produced from novel rubber impact modified dental liquid composition prepared in accordance with the invention blended with various commercially available denture polymer powders are characterized by improved impact resistance and flexural toughness. The impact resistance and flexural toughness of denture bases made from this novel impact modified dental liquids are greater than those using conventional denture base liquids commercially marketed at this time.

Furthermore, denture bases produced from novel rubber impact modified liquid compositions of the invention have excellent stain, chemical and solvent resistances. They also have excellent bonding strength to acrylic plastic teeth in the market. In comparison with conventional acrylic denture bases, the denture bases produced in accordance with the invention are characterized by outstanding impact resistance, and improved flexural toughness.

The novel rubber impact modified liquid compositions are formed in accordance with the invention by combining at least a monomer, crosslinking agents for said monomer, at least a rubber impact modifier, which disperses evenly and maintains a homogeneous appearance in this liquid.

Monomer compounds that can be used in the composition of this invention are methyl methacrylate, methyl acrylate, ethyl methacrylate, isobutyl methacrylate, cyclohexyl methacrylate, isobornyl methacrylate, isobornyl acrylate, and allyl methacrylate. A plurality of different monomer compounds may be included to control polymerization shrinkage, polymerization rate, mixing consistency and working time after mixed with polymer powders, and physical and mechanical properties after cured.

Crosslinking agents that can be used in the composition of this invention are di- or poly-acrylates and methacrylates such as glycerol di(meth)acrylate, glycerol tri(meth)acrylate, ethyleneglycol di(meth)acrylate, diethyleneglycol di(meth)acrylate, triethyleneglycol dimethacrylate, tetraethylene glycol di(meth)acrylate, poly(ethylene glycol) di(meth)acrylate, poly(propylene glycol) di(meth)acrylate, 1,3-propanediol di(meth)acrylate, 1,3-propanediol di(meth)acrylate, 1,4-butanediol di(meth)acrylate, 1,3- butylene glycol di(meth)acrylate, trimethylolpropane tri(meth)acrylate, 1,2,4-butanetriol trimethacrylate, 1,4-cyclohexanediol diacrylate, 1,4-cyclohexanediol dimethacrylate, 1,6-hexanediol di(meth)acrylate, neopentyl glycol di(meth)acrylate, tris(2-hydroxy ethyl)isocyanurate diacrylate, tris(2-hydroxy ethyl)isocyanurate triacrylate, pentaerythritol tri(meth)acrylate, pentaerythritol tetra(meth)acrylate, pentaerythritol tetramethacrylate, sorbitol hexacrylate, 2,2-bis[4-(2-hydroxy-3-acryloyloxypropoxy)phenyl]propane; 2,2-bis[4-(2-hydroxy-3-methacryloyloxypropoxy)phenyl]propane (Bis-GMA); 2,2-bis[4-(acryloyloxy-ethoxy)phenyl]propane; 2,2-bis[4-(methacryloyloxy-ethoxy)phenyl]propane (or ethoxylated bisphenol A-dimethacrylate) (EBPADMA); urethane di(meth)acrylate (UDMA), such as urethane di(meth)acrylate derivative of (isocyanatomethyl)cyclohexane (e.g., 1,3-bis(isocyanatomethyl)cyclohexane), UCDPMAA of example 2, diurethane dimethacrylate (DUDMA), 4,13-dioxo-3,14 dioxa-5,12-diazahexadecane-1,16-diol diacrylate; 4,13-dioxo-3,14 dioxa-5,12-diazahexadecane-1,16-diol dimethacrylate; the reaction product of trimethyl 1,6-diisocyanatohexane and bisphenol A propoxylate and 2-hydroxyethyl methacrylate (TBDMA); the reaction product of 1,6 diisocyanatohexane and 2-hydroxyethyl methacrylate modified with water (HDIDMA); the reaction product of 1,6 diisocyanatohexane and 2-hydroxyethyl acrylate modified with water (HDIDA); polyurethane dimethacrylate (PUDMA); alkoxylated pentaerythritol tetraacrylate; polycarbonate dimethacrylate (PCDMA); the bis-acrylates and bis-methacrylates of polyethylene glycols; copolymerizable mixtures of acrylated monomers and acrylated oligomers and combinations thereof.

The activatable material includes at least one impact modifier. As used herein, like with any other ingredients of the present invention, the term "impact modifier" can include one impact modifier or plural impact modifiers. Various impact modifiers may be employed in the practice of the present invention and often include one or more elastomers. The polymerizable liquid component includes 1 to 15 weight percent rubber impact modifier.

The impact modified composition may optionally include one or more additives that can include, without limitation, at least one filler (e.g., fibers, glass particles or otherwise), an initiator, a catalyst, an accelerator, an inhibitor, surfactant, or combinations thereof.

Examples of suitable fillers include, but are not limited to polyester fibers, nylon fibers, red acetate fibers, highly crosslinked polymer beads, silanized glass, silica beads, and combinations thereof.

Examples of initiators, include, but are not limited to, dibenzoyl peroxide (BPO), dilauroyl peroxide (LPO), t-butylhydroperoxide, cumene hydroperoxide, di-t-butyl peroxide, dicumyl peroxide, acetyl peroxide, 1-benzyl-5-phenylbarbituric acid (PBS), 5-n-butylbarbituric acid (BBS), an organic peroxide and an amine, an amine and a sulfinic acid salt, an acidic compound and an aryl borate, barbituric acid and alkylborane, barbituric acid and alkyl ammonium chloride/copper chloride, 2,2'-azobis-(isobutyronitrile) (AIBN), 2,2'-azobis-(2,4-dimethyl valeronitrile) (ADMV), tert-butyl per-2-ethyhexanoate (t-BPEH), and combinations thereof. Other initiating components may include, but are not limited to room temperature or heat activating catalyst components (e.g., system) for curing polymerizable materials (e.g., dental materials) of the invention. For example a peroxide capable of producing free radicals when activated by a reducing agent at room temperature or by heating. Room temperature activated polymerization initiating compounds may preferably include the combinations of peroxide and amine, barbituric acid and copper and chloride ions. Heat-activated polymerization initiating compounds may be included to provide a heat-curable polymerizable material. The peroxides generate free radicals to initiate polymerization and hardening of the composition at elevated temperature. Peroxides such as dibenzoyl peroxide (BPO), di-p-chlorobenzoyl peroxide, di-2,4-dichlorobenzoyl peroxide, tertiary butyl peroxybenzoate, methyl ethyl ketone peroxide, ditertiary butyl peroxide, dicumyl peroxide and cumene hydroperoxide, and the like can be used.

Example of stabilizers include, but are not limited to, hydroquinone (HQ), 2,6-ditert-butyl 4-methyphenol (BHT), monomethyl ether hydroquinone (MeHQ), benzoquinone, chloranil, phenol, butyl hydroxyanaline, tertiary butyl hydroquinone (TBHQ), tocopherol (Vitamin E), and the like. Preferably, butylated hydroxytoluene (BHT) and the methyl ether of hydroquinone (MEHQ) are used as the stabilizers (polymerization inhibitors). Other stabilizers, such UV absorbers, may also be used.

The rubber impact modifiers are in the form of small particles having average diameters ranging from about 0.01 micron to about 200 microns. Preferably, particles have diameters ranging from 0.02 micron to about 20 microns. More preferably, particles have diameters ranging from 0.05 micron to about 10 micron. The rubber impact modifiers particles are fully dispersed into the monomer, crosslinking agents and the rest of liquid. Its hard shells are fully swollen and penetrated by the used monomer while the soft cores remain relative intact so as to maintain distinct hard and soft phases and provide adequate suspension in the rest of components in composition and become a part of crosslinked and interpenetrating polymer network. It has been discovered that the composition of this rubber impact modifier and relative proportion of this modifier dramatically affect the impact resistance and fracture toughness of final cured composition as well as the handling properties at uncured stage. This invention provides components for a desired composition to the attainment of the desired properties in the final hardened or cured product produced therefrom, notably the impact resistance and fracture toughness.

It has been discovered that the relative proportions of the components of the rubber impact modified liquids produced in accordance with the invention are critical to the attainment of the desired handling properties at uncured stage. It has been discovered that the relative proportions of the components of the rubber impact modified liquids produced in accordance with the invention are critical to the attainment of the desired properties in the final hardened or cured product produced therefrom, notably the wear resistance, bond strength, flexural properties, impact strength, fracture toughness, resistance to MMA monomer and other solvents, stain resistance, and hydrolytic stability. The rubber impact modified compositions (e.g., liquid compositions) include 1 to 15 weight percent of rubber impact modifiers, 50 to 98 weight percent of at least a polymerizable monomer, and 1 to 49 weight percent of crosslinked agents for said at least a polymerizable monomer. Optionally, the impact modified composition may further include minor amounts of other ingredients, such as emulsifiers or/and surfactants, inorganic or/and polymer fillers, initiator and/or in some cases an activator for the initiator. The present invention may provide for rubber impact modified compositions, which are particularly useful in the production of acrylic denture bases with properties, especially impact resistance and fracture toughness, superior to those of conventional acrylic systems now used in the art. Advantageously, the impact modified compositions of the present invention provide for the introduction of unique homogeneous rubber impact modified liquids, rubber impact modifier, which enhanced the impact strength and fracture toughness of cured product surprisingly.

As used herein, the term core/shell impact modifier may denote an impact modifier wherein a substantial portion (e.g., greater than 30%, 50%, 70% or more by weight) thereof is comprised of a first polymeric material (i.e., the first or core material) that is substantially entirely encapsulated by a second polymeric material (i.e., the second or shell material). The first and second polymeric materials, as used herein, can be comprised of one, two, three or more polymers that are combined and/or reacted together (e.g., sequentially polymerized) or may be part of separate or same core/shell systems.

The first and second polymeric materials of the core/shell impact modifier can include elastomers, polymers, thermoplastics, copolymers, other components, combinations thereof or the like. In preferred embodiments, the first polymeric material, the second polymeric material or both of the core/shell impact modifier include or are substantially entirely composed of (e.g., at least 70%, 80%, 90% or more by weight) one or more thermoplastics. Exemplary thermoplastics include, without limitation, polycarbonate, polyester, polyolefin, polystyrene polypropylene, polyethylene terephthalate, polyvinyl chloride, polyamide, polyethylene, polybutylene terephthalate, acrylonitrile-butadiene-styrene resin, polymethyl methacrylate, or the like, and/or any combination thereof. According to the invention, butadiene-based rubber (e.g., MMA-butadiene-styrene or Acrylonitrile-butadiene-styrene) core/shell impact modifiers are used to achieve both superior high impact strength and/or excellent weatherability.

Preferred core/shell impact modifiers are formed by emulsion polymerization followed by coagulation or spray drying. It is also preferred for the impact modifier to be formed of or at least include a core-shell graft co-polymer. The first or core polymeric material of the graft copolymer preferably has a glass transition temperature substantially below (i.e., at least 10, 20, 40 or more degrees centigrade) the glass transition temperature of the second or shell polymeric material. Moreover, it may be desirable for the glass transition temperature of the first or core polymeric material to be below 23°C. (e.g., below 10°C) while the glass transition temperature of the second or shell polymeric material to be above 23° C., although not required.

Examples of useful core-shell graft copolymers are those where hard containing compounds, such as styrene, acrylonitrile or methyl methacrylate, are grafted onto core made from polymers of soft or elastomeric containing butadiene. The core polymer, may also include other copolymerizable containing compounds, such as styrene, vinyl acetate, methyl methacrylate, isoprene, or the like. The core polymer material may also include a cross linking monomer having two or more nonconjugated double bonds of approximately equal reactivity such as ethylene glycol diacrylate, butylene glycol dimethacrylate, and the like. The core polymer material may also include a graft linking monomer having two or more nonconjugated double bonds of unequal reactivity.

A characteristic of the rubber impact modified liquid is that the rubber impact modifier will be insoluble in, but will absorb or imbibe, the liquid polymerizable monomer component used in the preparation of the rubber impact modified liquid and form a colloid, a homogeneous mixture. Rubber impact modifiers can be used in the composition of this invention, include, but are not limited to, Metablen S2006, S2001, S2030, SRK200, C223 (all sold from Mitsubishi Rayon Co.), and D440 (sold by Arkema), etc.

Many prior arts used rubber impact modifiers in polymer powder synthesis to enhance the impact resistance and fracture toughness of denture bases, which also significantly affect the manufacturing process of conventional PMMA polymer powder since the rubber impact modifiers are needed to incorporate into polymer. This invention used a novel and simple approach by incorporating rubber impact modifier particles into liquid to enhance the impact resistance and fracture toughness of final cured denture base.

Generally, the present invention may include a composition having a composition as shown in Table 1.

**Table 1 - Impact Modified Composition**

| Component | Wt% Range (Preferred) |
|---|---|
| Crosslinker(s) | 1 - 15 |
| Monomer(s) | 50 - 95 (e.g., 70 - 95) |
| Rubber Impact Modifier(s) | 2 - 25 (e.g., 3 - 15) |
| Initiator(s) | 0.05 - 2 |
| Stabilizer(s) | 0.005 - 3 |
| Inhibitor(s) | .0005 - 1 |
| Filler(s) | 0 - 15 |
| Other(s) | 0 - 5 |

### EXAMPLE 1

### Preparation of Oligomer (TBDMA)

A reactor was charged with 1176 grams of trimethyl-1,6-diisocyanatohexane (5.59 mol) and 1064 grams of bisphenol A propoxylate (3.09 mol) under dry nitrogen flow and heated to about 65°C under positive nitrogen pressure. To this reaction mixture, 10 drops of catalyst dibutyltin dilaurate were added. The temperature of the reaction mixture was maintained between 65°C and 140°C for about 70 minutes and followed by additional 10 drops of catalyst dibutyltin dilaurate. A viscous paste-like isocyanate end-capped intermediate product was formed and stirred for 100 minutes. To this intermediate product, 662 grams (5.09 mol) of 2-hydroxyethyl methacrylate and 7.0 grams of BHT as an inhibitor were added over a period of 70 minutes while the reaction temperature was maintained between 68°C and 90°C. After about five hours stirring under 70°C, the heat was turned off, and oligomer was collected from the reactor as semi-translucent flexible solid and stored in a dry atmosphere.

### EXAMPLE 2

### Preparation of Urethane Monomer (UCDPMAA)

A 500 mL flask was charged with 38.8 grams (0.200 mol) of 1,3-bis(isocyanatomethyl)cyclohexane under dry nitrogen flow and heated to about 60°C under positive nitrogen pressure. To this reaction mixture, 3 drops of catalyst dibutyltin dilaurate were added. A mixture of 22.7 grams of 2-hydroxy-3-phenoxy propyl acrylate, 26.6 grams (0.204 mol) of 2-hydroxyethyl methacrylate, 11.5 grams (0.099 mol) of 2-hydroxyethyl acrylate and 0.10 grams of BHT as an inhibitor were added over a period of 70 minutes while the reaction temperature was maintained between 56°C and 78°C. After about four hours stirring, the heat was turned off, and monomer was collected from the flask as viscous liquid and stored in a dry atmosphere.

### Rubber impact modified liquids

Rubber impact modified liquids were used to mix with the polymer powders to form pourable liquids or packable doughs to fabricate dental devices. Rubber impact modified liquids contain at least one methacrylate or acrylate monomers and crosslinkers. This liquid mixed with polymer powder produced material with desirable handing properties for pourable or packable applications, which cured to form high impact strength dental material with superior fracture toughness. The impact modified liquids of examples 3-10 are not according to the invention, but for reference only.

### EXAMPLE 3

### Impact modified liquids

A polymerizable dental material was prepared by stirring at room temperature a liquid mixture of 5 grams of S2006 (from Mitsubishi Rayon), 20 grams of oligomer (TBDMA) made following the procedure of Example 1; 2 grams of ethylene glycol dimethacrylate (EGDMA); 73 grams of methyl methacrylate (MMA).

### EXAMPLE 4

### Impact modified liquids

A polymerizable dental material was prepared by stirring at room temperature a liquid mixture of 3 grams of S2006 (from Mitsubishi Rayon), 2 grams of monomer (UCDPMAA) made following the procedure of Example 2; 2 grams of ethylene glycol dimethacrylate (EGDMA); 93 grams of methyl methacrylate (MMA) containing initiating package..

### EXAMPLE 5

### Impact modified liquids

A polymerizable dental material was prepared by stirring at room temperature a liquid mixture of 7.5 grams of S2006 (from Mitsubishi Rayon); 3 grams of tris(2-hydroxy ethyl)isocyanurate diacrylate (THEICDA); 89.5 grams of methyl methacrylate (MMA) containing initiating package..

### EXAMPLE 6

### Impact modified liquids

A polymerizable dental material was prepared by stirring at room temperature a liquid mixture of 6 grams of S2006 (from Mitsubishi Rayon); 3 grams of tris(2-hydroxy ethyl)isocyanurate diacrylate (THEICDA); 81 grams of methyl methacrylate (MMA) containing initiating package; and 10 grams of butyl diglycol methacrylate.

### EXAMPLE 7

### Impact modified liquids

A polymerizable dental material was prepared by stirring at room temperature a liquid mixture of 6 grams of S2006 (from Mitsubishi Rayon); 5 grams of tris(2-hydroxy ethyl)isocyanurate diacrylate (THEICDA); 75 grams of methyl methacrylate (MMA) containing initiating package; and 14 grams of 2-phenoxyethyl methacrylate.

### EXAMPLE 8

### Impact modified liquids

A polymerizable dental material was prepared by stirring at room temperature a liquid mixture of 6.5 grams of S2006 (from Mitsubishi Rayon); 93.5 grams of Lucitone 199 liquid (sold by Dentsply International).

### EXAMPLE 9

### Impact modified liquids

A polymerizable dental material was prepared by stirring at room temperature a liquid mixture of 5 grams of S2006 (from Mitsubishi Rayon); 95 grams of Lucitone 199 liquid (sold by Dentsply International).

### EXAMPLE 10

### Impact modified liquids

A polymerizable dental material was prepared by stirring at room temperature a liquid mixture of 6.5 grams of S2006 (from Mitsubishi Rayon); 25 grams of oligomer (TBDMA) made according to Example 1; 68.5 grams of methyl methacrylate (MMA) liquid (from Lucite).

### Polymer powders

Polymer powders were used to mix with the impact modified liquid to form pourable liquids or packable doughs to fabricate dental devices. Polymer powders contain at least one selected from methyl methacrylate based polymers, copolymers, crosslinked polymers, plasticized polymers, rubber impact modified polymers or their combinations. These polymer powders mixed with impact modified liquids produced materials with desirable handing properties for pourable or packable applications, which cured to form high impact strength dental materials with superior fracture toughness.

### EXAMPLE 11

### Polymer powders

A polymer powder was prepared by mixing 75% Colacryl TS 1785 (from Lucite International) and 25% Colacryl TS 1785 (from Lucite International) containing milled pigments and initiators.

### EXAMPLE 12

### Polymer powders

A polymer powder was prepared by mixing 40% Colacryl TS 1785 (from Lucite International)), 35% DA441 (from Makevale Group) and 25% Colacryl TS 1785 containing milled pigments and initiators.

### EXAMPLE 13

### Polymer powders

A polymer powder was prepared by mixing 5% Colacryl TS 1785 (from Lucite International), 30% plasticized polymer (from Esstech, Inc.), 40% DA441 (from Makevale Group) and 25% Colacryl TS 1785 containing milled pigments and initiators.

### EXAMPLE 14

### Polymer powders

A polymer powder was prepared by mixing 40% plasticized polymer (from Esstech, Inc.), 40% DA442 (from Makevale Group) and 20% plasticized polymer (from Esstech, Inc.) containing milled pigments.

### Dental material mixtures

Polymerizable dental material mixtures were prepared by mixing polymer powders and rubber impact modified liquids to form pourable liquids or packable doughs for subsequent fabrication of dental devices. Pourable liquids were used with pouring or injection techniques to fabricate dental devices. Packable doughs were used by packing or injection techniques and heat/cold cured to form dental devices. The dental material mixtures of examples 15-22 are not according to the invention, but for reference only.

### EXAMPLE 15

### Pourable acrylics

A pour acrylic mixture was prepared by mixing 20 grams of polymer powders of Example 11 and 14 grams of liquid of Example 6. This mixture was poured into a previously prepared flask and bench set for 5 minutes and then cured in 55°C pressure pot for 30 minutes. After cure, a full denture was removed. After finished and polished, it could be delivered to the patient.

### EXAMPLE 16

### Pourable acrylics

A pour acrylic mixture was prepared by mixing 14.33 grams of polymer powders of Example 11 and 10 grams of liquid of Example 6. This mixture was poured into a previously prepared flask with silicone mold and bench set for 5 minutes and then cured in 40°C pressure pot for 30 minutes. After cure, bar sample was removed. After cutting into testing specimen and finished, it was hydrated for 7 days in 37°C water bath and tested for fracture toughness according to ISO 20795-1. With the similar procedure, flexural strength testing specimen were prepared, hydrated and tested according to ISO 20795-1

### EXAMPLE 17

### Pourable acrylics

A pour acrylic mixture was prepared by mixing 20 grams of polymer powders of Example 12 and 14 grams of liquid of Example 4. This mixture was poured into a previously prepared flask with hydrocolloid mold and bench set for 5 minutes and then cured in 40°C pressure pot for 30 minutes. After cure, bar sample was removed. After cutting into testing specimen and finished, it was hydrated for 7 days in 37°C water bath and tested for fracture toughness according to ISO 20795-1.

### EXAMPLE 18

### Pourable acrylics

A pour acrylic mixture was prepared by mixing 20 grams of polymer powders of Example 12 and 13 grams of liquid of Lucitone Fas-Por+ (sold by Dentsply International). This mixture was poured into a previously prepared flask with hydrocolloid mold and bench set for 5 minutes and then cured in 40°C pressure pot for 30 minutes. After cure, bar sample was removed. After cutting into testing specimen and finished, it was hydrated for 7 days in 37°C water bath and tested for fracture toughness according to ISO 20795-1.

### EXAMPLE 19

### Packable acrylics

A packable acrylic mixture was prepared by mixing 21 grams of polymer powders of Lucitone Clear (sold by Dentsply International) and 10 mL of liquid of Example 9. This mixture was packed into a previously prepared flask and cured for 90 minutes at 163°F and then 30 minutes boiling. After cure, bar sample was removed. After cutting into testing specimen and finished, it was hydrated for 7 days in 37°C water bath and tested for fracture toughness according to ISO 20795-1. With the similar procedure, flexural strength testing specimen were prepared, hydrated and tested according to ASTM 790 (1997).

### EXAMPLE 20

### Packable acrylics

A packable acrylic mixture was prepared by mixing 21 grams of polymer powders of Lucitone Clear (sold by Dentsply International) and 10 mL of liquid of Example 8. This mixture was packed into a previously prepared flask and cured for 90 minutes at 163°F and then 30 minutes boiling. After cure, bar sample was removed. After cutting into testing specimen and finished, it was hydrated for 7 days in 37°C water bath and tested for fracture toughness according to ISO 20795-1. With the similar procedure, flexural strength testing specimen were prepared, hydrated and tested according to ASTM 790 (1997).

### EXAMPLE 21

### Packable acrylics

A packable acrylic mixture was prepared by mixing 21 grams of polymer powders of Example 14 and 10 mL of liquid of Example 9. This mixture was packed into a previously prepared flask and cured for 90 minutes at 163°F and then 30 minutes boiling. After cure, bar sample was removed. After cutting into testing specimen and finished, it was hydrated for 7 days in 37°C water bath and tested for fracture toughness according to ISO 20795-1. With the similar procedure, flexural strength testing specimen were prepared, hydrated and tested according to ASTM 790 (1997).

### EXAMPLE 22

### Packable acrylics

A packable acrylic mixture was prepared by mixing 21 grams of polymer powders of Example 14 and 10 mL of liquid of Lucitone 199 (sold by Dentsply International). This mixture was packed into a previously prepared flask and cured for 90 minutes at 163°F and then 30 minutes boiling. After cure, bar sample was removed. After cutting into testing specimen and finished, it was hydrated for 7 days in 37°C water bath and tested for fracture toughness according to ISO 20795-1. With the similar procedure, flexural strength testing specimen were prepared, hydrated and tested according to ASTM 790 (1997).

It is appreciated that the impact modified compositions of the present invention may include a Kₘₐₓ of at least 1.8 (e.g., 1.9), preferably at least 2.0 (e.g., 2.1), and more preferably at least 2.3 MPa*m^{1/2}. Furthermore, the impact modified compositions of the present invention may include a Kₘₐₓ of less than 5, typically less than 4, and more typically less than 3.5 MPa*m^{1/2}. For example, the impact modified compositions of the present invention may include a Kₘₐₓ ranging from about 1.8 (e.g., about 1.9) to about 5, typically from about 2.0 (e.g., about 2.1) to about 4, and more typically from about 2.3 to about 3.5 MPa*m^{1/2}.

It is also appreciated that the impact modified compositions of the present invention may include a Work of Fracture of at least 700 (e.g., 900), preferably at least 1100 (e.g., 1500), and more preferably at least 2000 J/m². Furthermore, the impact modified compositions of the present invention may include a Work of Fracture of less than 10,000, typically less than 8,000, and more typically less than 6,000 J/m². For example, the impact modified compositions of the present invention may include a Work of Fracture ranging from about 700 to about 10,000, typically from about 1100 to about 8000, and more typically from about 2,000 to about 6,000 J/m².

Table 2 showed the flexural strength and fracture toughness of various acrylic denture formulations. Example 18 is a pour acrylic using currently commercially available cold cure (Lucitone Fas-Por+) liquid mixed with polymer powder of Example 12 (row 3 of Table 2). Lucitone Fas-Por+ (liquid and powder) is a commercially available product sold by Dentsply International. Both of these two materials gave relative low Kₘₐₓ and low Work of Fracture (rows 3 and 4 of Table 2). The use of rubber impact modified liquid gave surprising high fracture toughness as compared to commercially available dental cold cure acrylic compositions. Examples 16 and 17 used a rubber impact modified liquid, not according to this invention, which mixed with the same polymer powder of Example 12, gave improved Kₘₐₓ and significant higher Work of Fracture (rows 1 and 2 of Table 2). For heat cured acrylics, the use of acrylic liquid which improved fracture toughness significantly is shown in Examples 19 to 22 (rows 5 to 9 of Table 2). Lucitone Clear is a commercially available product sold by Dentsply International, the use of rubber impact modified liquids (Examples 19 and 20) not according to this invention to replace Lucitone Clear liquid improved Kₘₐₓ from 1.80 MPa*m ^{½} to 2.23 and 2.33 MPa*m ^{½}, and Work of Fracture from 380 J/m ² to 800 and 890 J/m ² respectively (increased from row 7 to rows 5 and 6 of Table 2). In addition, the use of rubber impact modified liquid not according to this invention also improved high impact rubber impact modified polymer based acrylics as shown in Example 21 and 22 (rows 8 and 9 of Table 2). Example 21 used rubber impact modified liquid further enhanced the fracture toughness of rubber impact modified polymer based system as compared to Example 22 (used liquid without rubber impact modifier).

Flexural Strength and Flexural Modulus of the polymerized acrylic compositions of Examples 19 to 22 and commercially available denture base materials (Lucitone Clear and Lucitone Fas-Por+) were measured by using three-point bend test on Instron bending unit according to ASTM 790 (1997) and ISO 20795-1:2008 (E). Fracture toughness of the polymerized acrylic compositions of Examples 16 to 22 and commercialized available denture base materials were measured by using a modified three-point bend test on Instron bending unit according to ISO 20795-1:2008 (E). Samples were prepared according to ISO 20795-1:2008 (E) and cured in flasks containing silicone and stone molds in pressure pot or water tank. Others were cured according to manufacturing instructions.

**Table 2. Fracture toughness and flexural strength of various formulations**

| | **Examples** | Flexural Strength (Psi) | Flexural Modulus (KPsi) | Kₘₐₓ (MPa*m ^{1/2}) | Work of Fracture (J/m²) |
|---|---|---|---|---|---|
| 1 | Example 16 | (67 MPa according to ISO 20795-1) | (2210 MPa according to ISO 20795-1) | 2.51 s.d. = 0.08 | 1160 s.d. = 100 |
| 2 | Example 17 | | | 2.32 s.d. = 0.1 0 | 1000 s.d. = 30 |
| 3 | Example 18 | | | 2.07 s.d. = 0.22 | 445 s.d. = 110 |
| 4 | Lucitone Fas-Por+ | 13,200 (69 MPa according to ISO 20795-1) | 400 (2320 MPa according to ISO 20795-1) | 1.83 s.d. = 0.08 | 330 s.d. = 20 |
| 5 | Example 19 | 13,700 | 390 | 2.23 s.d. = 0.10 | 800 s.d. = 40 |
| 6 | Example 20 | 13,400 | 390 | 2.33 s.d. = 0.04 | 890 s.d. = 30 |
| 7 | Lucitone Clear | 14,200 | 410 | 1.80 s.d. = 0.20 | 380 s.d. = 80 |
| 8 | Example 21 | 12,500 | 380 | 2.7 s.d. = 0.06 | 1530 s.d. = 20 |
| 9 | Example 22 | 13,300 | 380 | 2.5 s.d. = 0.06 | 1270 s.d. = 20 |

All examples are not according to the invention, but for reference only.

Lucitone Fas-Por+ (15 minutes at 45°C), and Lucitone Clear specimens were prepared according to manufacture instructions and tested according to ISO 20795-1 for fracture toughness and according to ASTM 790 (1997) for flexural property. Flexural strength of Example 16 was tested according to ISO 20795-1 and Flexural strength of Lucitone Fas-Por+ was also tested according to ISO 20795-1 and shown in bracket in above table.

It should be understood that while the present invention has been described in considerable detail with respect to certain specific embodiments thereof, it should not be considered limited to such embodiments but may be used in other ways without departure from the scope of the appended claims.

It will be further appreciated that functions or structures of a plurality of components or steps may be combined into a single component or step, or the functions or structures of one-step or component may be split among plural steps or components. The present invention contemplates all of these combinations. Unless stated otherwise, dimensions and geometries of the various structures depicted herein are not intended to be restrictive of the invention, and other dimensions or geometries are possible. In addition, while a feature of the present invention may have been described in the context of only one of the illustrated embodiments, such feature may be combined with one or more other features of other embodiments, within the scope of the claims. The use of "comprising" or "including" also contemplates embodiments that "consist essentially of" or "consist of" the recited feature.

The explanations and illustrations presented herein are intended to acquaint others skilled in the art with the invention, its principles, and its practical application. Those skilled in the art may adapt and apply the invention in its numerous forms, as may be best suited to the requirements of a particular use. Accordingly, the specific embodiments of the present invention as set forth are not intended as being exhaustive or limiting of the invention. The scope of the invention should, therefore, be determined not with reference to the above description, but should instead be determined with reference to the appended claims, along with the full scope to which such claims are entitled.

## Claims

1. A dental composition comprising
at least one polymerizable liquid component including at least one rubber impact modifier;
at least one polymerization initiator, and
at least one polymer powder component,
wherein the resultant composition forms a flowable liquid,
wherein the at least one polymerizable liquid component includes 50 to 98 weight percent of monomer, 1 to 49 weight percent of crosslinking agent for the monomer, and 1 to 15 weight percent rubber impact modifier,
wherein the at least one monomer is selected from a group consisting of methyl methacrylate, methyl acrylate, ethyl methacrylate, isobutyl methacrylate, cyclohexyl methacrylate, isobornyl methacrylate, isobornyl acrylate, and allyl methacrylate,
wherein the at least one crosslinking agent is selected from a group consisting of di- or poly-acrylates and methacrylates, and
wherein the rubber impact modifier is a butadiene-based core-shell rubber impact modifier which does not dissolve but swells and forms a colloid in the at least one polymerizable liquid component.

2. The composition of claim 1, wherein the at least one crosslinking agent is selected from a group consisting of glycerol di(meth)acrylate, glycerol tri(meth)acrylate, ethyleneglycol di(meth)acrylate, diethyleneglycol di(meth)acrylate, triethyleneglycol dimethacrylate, tetraethylene glycol di(meth)acrylate, poly(ethylene glycol) di(meth)acrylate, poly(propylene glycol) di(meth)acrylate, 1,3-propanediol di(meth)acrylate, 1,3- propanediol di(meth)acrylate, 1,4-butanediol di(meth)acrylate, 1,3- butylene glycol di(meth)acrylate, trimethylolpropane tri(meth)acrylate, 1,2,4-butanetriol trimethacrylate, 1,4- cyclohexanediol diacrylate, 1,4-cyclohexanediol dimethacrylate, 1,6-hexanediol di(meth)acrylate, neopentyl glycol di(meth)acrylate, tris(2-hydroxy ethyl)isocyanurate diacrylate, tris(2-hydroxy ethyl)isocyanurate triacrylate, pentaerythritol tri(meth)acrylate, pentaerythritol tetra(meth)acrylate, pentaerythritol tetramethacrylate, sorbitol hexacrylate, 2,2-bis[4-(2-hydroxy- 3-acryloyloxypropoxy)phenyl]propane; 2,2-bis[4-(2-hydroxy-3- methacryloyloxypropoxy)phenyl]propane (Bis-GMA); 2,2-bis[4-(acryloyloxy- ethoxy)phenyl]propane; 2,2-bis[4"(methacryloyloxy-ethoxy)phenyl]propane (or ethoxylated bisphenol A-dimethacrylate) (EBPAD A); urethane di(meth)acrylate (UDMA), such as urethane di(meth)acrylate derivative of (isocyanatomethyl)cyclohexane (e.g., 1,3-bis(isocyanatomethyl)cyclohexane), UCDPMAA, diurethane dimethacrylate (DUDMA), 4,13-dioxo-3,14 dioxa-5, 2-diazahexadecane-1,16-diol diacrylate; 4,13-dioxo-3,14 dioxa-5,12-diazahexadecane-1,16-diol dimethacrylate; the reaction product of trimethyl 1,6-diisocyanatohexane and bisphenol A propoxylate and 2-hydroxyethyl methacrylate (TBDMA); the reaction product of 1,6 diisocyanatohexane and 2-hydroxyethyl methacrylate modified with water (HDIDMA); the reaction product of 1,6 diisocyanatohexane and 2-hydroxyethyl acrylate modified with water (HDIDA); polyurethane dimethacrylate (PUDMA); alkoxylated pentaerythritol tetraacrylate; polycarbonate dimethacrylate (PCDMA); the bis-acrylates and bis-methacrylates of polyethylene glycols; copolymerizable mixtures of acrylated monomers and acrylated oligomers and combinations thereof.

3. The composition of claim 1, wherein the at least one impact modifier includes a core/shell impact modifier having greater than 30% by wt being a first polymeric core material that is encapsulated by a second polymeric shell material.

4. The composition of claim 3, wherein the first polymeric core material and the second polymeric shell material include one or more polymers that are combined and/or reacted together (e.g., sequentially polymerized) or may be part of separate or same core/shell systems.

5. The composition of claim 1, wherein the at least one monomer of the at least one polymerizable liquid component includes methyl methacrylate (MMA).

6. The composition of claim 1, further comprising one or more additives selected from the group consisting of at least one filler, an accelerator, an inhibitor, surfactant, and combinations thereof, wherein the at least one filler is preferably selected from the group consisting of fibers and glass particles.

## Patentansprüche

1. Dentalzusammensetzung,
zumindest eine polymerisierbare flüssige Komponente umfassend, die zumindest ein Schlagzähigkeitsmodifizierungsmittel aus Kautschuk, zumindest einen Polymerisationsinitiator und
zumindest eine Polymerpulverkomponente beinhaltet,
wobei die resultierende Zusammensetzung eine fließfähige Flüssigkeit bildet,
wobei die zumindest eine polymerisierbare flüssige Komponente 50 bis 98 Gew.-% Monomer, 1 bis 49 Gew.-% Vernetzungsmittel für das Monomer und 1 bis 15 Gew.-% Schlagzähigkeitsmodifizierungsmittel aus Kautschuk enthält, wobei das zumindest eine Monomer aus einer Gruppe ausgewählt ist, die aus Methylmethacrylat, Methylacrylat, Ethylmethacrylat, Isobutylmethacrylat, Cyclohexylmethacrylat, Isobornylmethacrylat, Isobornylacrylat, und Allylmethacrylat besteht,
wobei das zumindest eine Vernetzungsmittel aus einer Gruppe ausgewählt ist, die aus Di- oder Polyacrylaten und Methacrylaten besteht, und
wobei das Schlagzähigkeitsmodifizierungsmittel aus Kautschuk ein Kern-Schale-Schlagzähigkeitsmodifizierungsmittel aus Kautschuk auf Butadienbasis ist, das sich nicht löst, sondern aufquillt und ein Kolloid in der zumindest einen polymerisierbaren flüssigen Komponente bildet.

2. Zusammensetzung nach Anspruch 1, wobei das zumindest eine Vernetzungsmittel aus einer Gruppe ausgewählt ist, die aus Glycerindi(meth)acrylat, Glycerintri(meth)acrylat, Ethylenglykoldi(meth)acrylat, Diethylenglykoldi(meth)acrylat, Triethylenglykoldimethacrylat, Tetraethylenglykoldi(meth)acrylat, Poly(ethylenglykol)di(meth)acrylat, Poly(propylenglykol)di(meth)acrylat, 1,3-Propandioldi(meth)acrylat, 1,3-Propandiol-di(meth)acrylat, 1,4-Butandioldi(meth)acrylat, 1,3-Butylenglykoldi(meth)acrylat, Trimethylolpropantri(meth)acrylat, 1,2,4-Butantrioltrimethacrylat, 1,4-Cyclohexandioldiacrylat, 1,4-Cyclohexandioldimethacrylat, 1,6-Hexandioldi(meth)acrylat, Neopentylglykoldi(meth)acrylat, Tris(2-hydroxyethyl)isocyanuratdiacrylat, Tris(2-hydroxyethyl)isocyanurattriacrylat, Pentaerythritoltri(meth)acrylat, Pentaerythritoltetra(meth)acrylat, Pentaerythritoltetramethacrylat, Sorbitolhexacrylat, 2,2-Bis[4-(2-hydroxy-3-acryloyloxypropoxy)phenyl]propan; 2,2-Bis[4-(2-hydroxy-3-methacryloyloxypropoxy)phenyl]propan (Bis-GMA); 2,2-Bis[4-(acryloyloxy-ethoxy)phenyl]propan; 2,2-Bis[4"(methacryloyloxyethoxy)phenyl]propan (oder ethoxyliertem Bisphenol-A-dimethacrylat) (EBPAD A); Urethandi(meth)acrylat (UDMA), wie etwa Urethandi(meth)acrylatderivat von (Isocyanatomethyl)cyclohexan (z. B. 1,3-Bis(isocyanatomethyl)cyclohexan), UCDPMAA, Diurethandimethacrylat (DUDMA), 4,13-Dioxo-3,14 Dioxa-5, 2-Diazahexadecan-1,16-Dioldiacrylat; 4,13-Dioxo-3,14 Dioxa-5,12-Diazahexadecan-1,16-Dioldimethacrylat; das Reaktionsprodukt von Trimethyl 1,6-Diisocyanatohexan und Bisphenol A-Propoxylat und 2-Hydroxyethylmethacrylat (TBDMA); das Reaktionsprodukt von 1,6 Diisocyanatohexan und 2-Hydroxyethylmethacrylat modifiziert mit Wasser (HDIDMA); das Reaktionsprodukt von 1,6 Diisocyanatohexan und 2-Hydroxyethylacrylat modifiziert mit Wasser (HDIDA); Polyurethandimethacrylat (PUDMA); alkoxyliertes Pentaerythritoltetraacrylat; Polycarbonatdimethacrylat (PCDMA); die Bisacrylate und Bismethacrylate von Polyethylenglykolen; copolymerisierbare Mischungen von acrylierten Monomeren und acrylierten Oligomeren und Kombinationen davon.

3. Zusammensetzung nach Anspruch 1, wobei das zumindest eine Schlagzähigkeitsmodifizierungsmittel ein Kern/Schale-Schlagzähigkeitsmodifizierungsmittel mit mehr als 30 Gew.-% eines ersten polymeren Kernmaterials ist, das durch ein zweites polymeres Schalenmaterial eingekapselt ist.

4. Zusammensetzung nach Anspruch 3, wobei das erste polymere Kernmaterial und das zweite polymere Schalenmaterial ein oder mehrere Polymere beinhalten, die miteinander kombiniert und/oder umgesetzt werden (z. B. sequentiell polymerisiert) oder Teil von getrennten oder gleichen Kern/Schalen-Systemen sein können.

5. Zusammensetzung nach Anspruch 1, wobei das zumindest eine Monomer aus der zumindest Methylmethacrylat (MMA) beinhaltet.

6. Zusammensetzung nach Anspruch 1, ferner ein oder mehrere Additive umfassend, das/die aus der Gruppe ausgewählt ist/sind, die aus zumindest einem Füllstoff, einem Beschleuniger, einem Inhibitor, einem Tensid und Kombinationen davon besteht, wobei der zumindest eine Füllstoff bevorzugt aus der Gruppe ausgewählt ist, die aus Fasern und Glaspartikeln besteht.

## Revendications

1. Composition dentaire comprenant
au moins un composant liquide polymérisable comportant au moins un agent antichoc en caoutchouc ;
au moins un amorceur de polymérisation ; et
au moins un composant de poudre polymère,
dans laquelle la composition résultante forme un liquide fluide,
dans lequel l'au moins un composant liquide polymérisable comporte 50 à 98 pour cent en poids de monomère, de 1 à 49 pour cent en poids d'agent de réticulation pour le monomère, et de 1 à 15 pour cent en poids d'agent antichoc en caoutchouc,
dans laquelle l'au moins un monomère est choisi dans un groupe constitué du méthacrylate de méthyle, de l'acrylate de méthyle, du méthacrylate d'éthyle, du méthacrylate d'isobutyle, du méthacrylate de cyclohexyle, du méthacrylate d'isobornyle, de l'acrylate d'isobornyle et du méthacrylate d'allyle,
dans laquelle l'au moins un agent de réticulation est choisi dans un groupe constitué de di- ou poly-acrylates et de méthacrylates, et
dans laquelle l'agent antichoc en caoutchouc est un agent antichoc en caoutchouc cœur-écorce à base de butadiène qui ne se dissout pas, mais gonfle et forme un colloïde dans l'au moins un composant liquide polymérisable.

2. Composition selon la revendication 1, dans laquelle l'au moins un agent de réticulation est choisi dans un groupe constitué de di(méth)acrylate de glycérol, tri(méth)acrylate de glycérol, di(méth)acrylate d'éthylèneglycol, di(méth)acrylate de diéthylèneglycol, diméthacrylate de triéthylèneglycol, di(méth)acrylate de tétraéthylène glycol, di(méth)acrylate de poly(éthylène glycol), di(méth)acrylate de poly(propylène glycol), 1,3-propanediol di(méth)acrylate, 1,3-propanediol di(méth)acrylate, 1,4-butanediol di(méth)acrylate, 1,3-butylène glycol di(méth)acrylate, tri(méth)acrylate de triméthylolpropane, 1,2,4-butanetriol triméthacrylate, 1,4-cyclohexanediol diacrylate, 1,4-cyclohexanediol diméthacrylate, 1,6-hexanediol di(méth)acrylate, di(méth)acrylate de néopentylglycol, tris(2-hydroxyéthyl)isocyanurate diacrylate, tris(2-hydroxyéthyl)isocyanurate triacrylate, tri(méth)acrylate de pentaérythritol, tétra(méth)acrylate de pentaérythritol, tétraméthacrylate de pentaérythritol, hexacrylate de sorbitol, 2,2-bis[4-(2-hydroxy-3-acryloyloxypropoxy)phényl]propane ; 2,2-bis[4-(2-hydroxy-3-méthacryloyloxypropoxy)phényl]propane (Bis-GMA) ; 2,2-bis[4-(acryloyloxy-éthoxy)phényl]propane ; 2,2-bis[4"(méthacryloyloxy-éthoxy)phényl]propane (ou diméthacrylate de bisphénol A éthoxylé) (EBPAD A) ; uréthane di(méth)acrylate (UDMA), tel qu'un dérivé uréthane di(méth)acrylate de (isocyanatométhyl)cyclohexane (par exemple, 1,3-bis(isocyanatométhyl)cyclohexane), UCDPMAAA, diuréthane diméthacrylate (DUDMA), 4,13-dioxo-3,14 dioxa-5, 2-diazahexadécane-1,16-diol diacrylate ; 4,13-dioxo-3,14 dioxa-5,12-diazahexadécane-1,16-diol diméthacrylate ; le produit de réaction du triméthyle 1,6-diisocyanatohexane et du propoxylate de bisphénol A et du méthacrylate de 2-hydroxyéthyle (TBDMA) ; le produit de réaction de 1,6 diisocyanatohexane et du méthacrylate de 2-hydroxyéthyle modifié avec de l'eau (HDIDMA) ; le produit de réaction de 1,6 diisocyanatohexane et de l'acrylate de 2-hydroxyéthyle modifié avec de l'eau (HDIDA) ; diméthacrylate de polyuréthanne (PUDMA) ; tétraacrylate de pentaérythritol alcoxylé ; diméthacrylate de polycarbonate (PCDMA) ; bis-acrylates et bis-méthacrylates de polyéthylène glycols ; mélanges copolymérisables de monomères acrylés et d'oligomères acrylés et des combinaisons de ceux-ci.

3. Composition selon la revendication 1, dans laquelle l'au moins un agent antichoc comporte un agent antichoc cœur-écorce ayant plus de 30 % en poids qui est un premier matériau de cœur polymère qui est encapsulé par un second matériau d'écorce polymère.

4. Composition selon la revendication 3, dans laquelle le premier matériau de cœur polymère et le second matériau d'écorce polymère comportent un ou plusieurs polymères qui sont combinés et/ou mis à réagir ensemble (par exemple, polymérisés séquentiellement) ou peuvent faire partie de systèmes cœur-écorce séparés ou identiques.

5. Composition selon la revendication 1, dans laquelle l'au moins un monomère de l'au moins un composant liquide polymérisable comporte le méthacrylate de méthyle (MMA).

6. Composition selon la revendication 1, comprenant en outre un ou plusieurs additifs choisis dans le groupe constitué d'au moins une charge, un accélérateur, un inhibiteur, un tensioactif et des combinaisons de ceux-ci, dans laquelle l'au moins une charge est de préférence choisie dans le groupe constitué de fibres et de particules de verre.
